# FASCICULE DE BREVET EUROPEEN

(11) **EP 1 690 944 B1**
(45) Date de publication et mention de la délivrance du brevet: **02.11.2011**
(21) Numéro de dépôt: 06290147.5
(22) Date de dépôt: 20.01.2006
(51) Int. Cl.: C12P 7/10

(54) **Procédé de production d'enzymes cellulolytiques et hémicellulolytiques utilisant les résidus de distillation de fermentation éthanolique d'hydrolysats enzymatiques de materiaux (ligno-)cellulosiques**
Verfahren zur Herstellung von Cellulase-Enzymen aus Destillationsrückständen der Äthanol-Fermentation von enzymatischen Hydrolysaten aus (Ligno-)cellulosischem Material
Production of cellulase enzymes using the distillation residues from ethanolic fermentation of enzymatic hydrolysates from (ligno-)cellulosic materials

(30) Priorité: 09.02.2005 FR 0501371
(43) Date de publication de la demande: 16.08.2006
(73) Titulaire: Institut Français du Pétrole, 92852 Rueil Malmaison Cedex (FR)
(72) Inventeur: Warzywoda, Michel, 92500 Rueil Malmaison (FR); Ballerini, Daniel, 78100 St. Germain en Laye (FR); Monot, Frédéric, 92000 Nanterre (FR)

(56) Documents cités:
- WO-A-03/052055
- FR-A- 2 597 502
- US-A- 3 990 944
- US-A- 4 762 788
- US-A- 5 981 233
- GREGG DAVID J ET AL: "Factors affecting cellulose hydrolysis and the potential of enzyme recycle to enhance the efficiency of an integrated wood to ethanol process" BIOTECHNOLOGY AND BIOENGINEERING, vol. 51, no. 4, 1996, pages 375-383, XP002384205 ISSN: 0006-3592
- KIM JUN-SUK ET AL: "Ethanol production from lignocellulosic biomass by simultaneous saccharification and fermentation employing the reuse of yeast and enzyme" JOURNAL OF MICROBIOLOGY AND BIOTECHNOLOGY, vol. 9, no. 3, juin 1999 (1999-06), pages 297-302, XP008065049 ISSN: 1017-7825
- NAVARRO A R ET AL: "Bio-concentration of vinasse from the alcoholic fermentation of sugar cane molasses" WASTE MANAGEMENT, vol. 20, no. 7, 2000, pages 581-585, XP002384278 ISSN: 0956-053X
- RODRIGUEZ-LEON J A ET AL: "PRODUCTION OF SPORES OF TRICHODERMA HARZIANUM ON SUGAR CANE MOLASSES AND BAGASSE PITH IN SOLID STATE FERMENTATION FOR BIOCONTROL" BRAZILIAN ARCHIVES OF BIOLOGY AND TECHNOLOGY, INSTITUTO DE TECNOLOGIA DO PARANA, BR, vol. 42, no. 1, 1999, pages 69-76, XP008057364 ISSN: 1516-8913
- BOOPATHY RAMARAJ ET AL: "Microbial decomposition of post-harvest sugarcane residue" BIORESOURCE TECHNOLOGY, vol. 79, no. 1, août 2001 (2001-08), pages 29-33, XP002384342 ISSN: 0960-8524
- CHAHAL PARMINDER S ET AL: "Production of cellulase in solid-state fermentation with Trichoderma reesei MCG 80 on wheat straw" APPLIED BIOCHEMISTRY AND BIOTECHNOLOGY, vol. 57-58, no. 0, 1996, pages 433-442, XP008054568 ISSN: 0273-2289
- WARZYWODA M ET AL: "PRODUCTION AND CHARACTERIZATION OF CELLULOLYTIC ENZYMES FROM TRICHODERMA-REESEI GROWN ON VARIOUS CARBON SOURCES" BIORESOURCE TECHNOLOGY, vol. 39, no. 2, 1992, pages 125-130, XP008054567 ISSN: 0960-8524
- WINGREN ANDERS ET AL: "Techno-economic evaluation of producing ethanol from softwood: Comparison of SSF and SHF and identification of bottlenecks." BIOTECHNOLOGY PROGRESS, vol. 19, no. 4, 2003, pages 1109-1117, XP002384206 ISSN: 8756-7938
- FUJITA YASUYA ET AL: "Direct and efficient production of ethanol from cellulosic material with a yeast strain displaying cellulolytic enzymes", APPLIED AND ENVIRONMENTAL MICROBIOLOGY, vol. 68, no. 10, 2001, pages 5136-5141, XP002492142,
- GALBE M.; ZACCHI G: "A review for the production of ethanol from softwood", APPLIED MICROBIOLOGY AND BIOTECHNOLOGY, vol. 59, 2002, pages 618-628, XP002988032,
- STENBERG K. ET AL: "Recycling of process streams in ethanol production from softwoods based on enzymatic hydrolysis", APPLIED BIOCHEMISTRY AND BIOTECHNOLOGY, vol. 70-72, 1998, pages 697-708,
- ALKASRAWI M. ET AL: "Recirculation of process streams in fuel ethanol production from sooftwood based on simultaneous saccharification and fermentation", APPLIED BIOCHEMISTRY AND BIOTECHNOLOGY, vol. 98-100, 2002, pages 849-861,
- NUDEL B.C. ET AL: "The use of single and mixed cultures for aerobic treatment of cane sugae stillage and SCP production", BIOLOGICAL WASTES, vol. 22, 1987, pages 67-73,
- YANG F.-C.; LIN I.-H.: "Production of acid protease using thin stillage from a rice-spirit distillery by Aspergillus niger", ENZYME AND MICROBIAL TECHNOLOGY, vol. 23, 1998, pages 397-402,

## Description

La présente invention concerne la production d'enzymes cellulolytiques et/ou hémicellulolytiques, dans le cadre de la production d'éthanol à partir de matériaux cellulosiques ou ligno-cellulosiques, dans lequel le résidu constitué des vinasses de distillation est utilisé, après séparation de l'éthanol, comme source de carbone inductrice ou totale pour la production des enzymes cellulolytiques.

Depuis les années 1970, la transformation de la biomasse ligno-cellulosique en éthanol, après hydrolyse des polysaccharides constitutifs en sucres fermentescibles, a fait l'objet de nombreux travaux.

Les bois de feuillus et les pailles de céréales sont les substrats les plus utilisés. Ils sont constitués pour la plupart d'environ 40 à 50 % de cellulose, 20 à 25 % d'hémicellulose et de 15 à 25 % de lignine.

D'autres ressources, cultures forestières dédiées, résidus de plantes alcooligènes, sucrières et céréalières, résidus de l'industrie papetière et des produits de transformation des matériaux cellulosiques et ligno-cellulosiques sont utilisables.

De ces trois polymères, cellulose, hémicellulose et lignine, la cellulose est la principale source de sucres fermentescibles en éthanol car elle est constituée de glucose, ce dernier étant facilement fermenté en éthanol par *Saccharomyces cerevisiae* dans des procédés industriels éprouvés et performants. Les pentoses contenus dans les hémicelluloses ne sont pas efficacement convertis en éthanol. D'autres microorganismes parmi les genres *Saccharomyces, Pichia, Candida, Pachysolen, Zymomonas, Klebsiella, Escherichia,* peuvent être choisis pour valoriser les sucres monomères issus de la biomasse en éthanol.

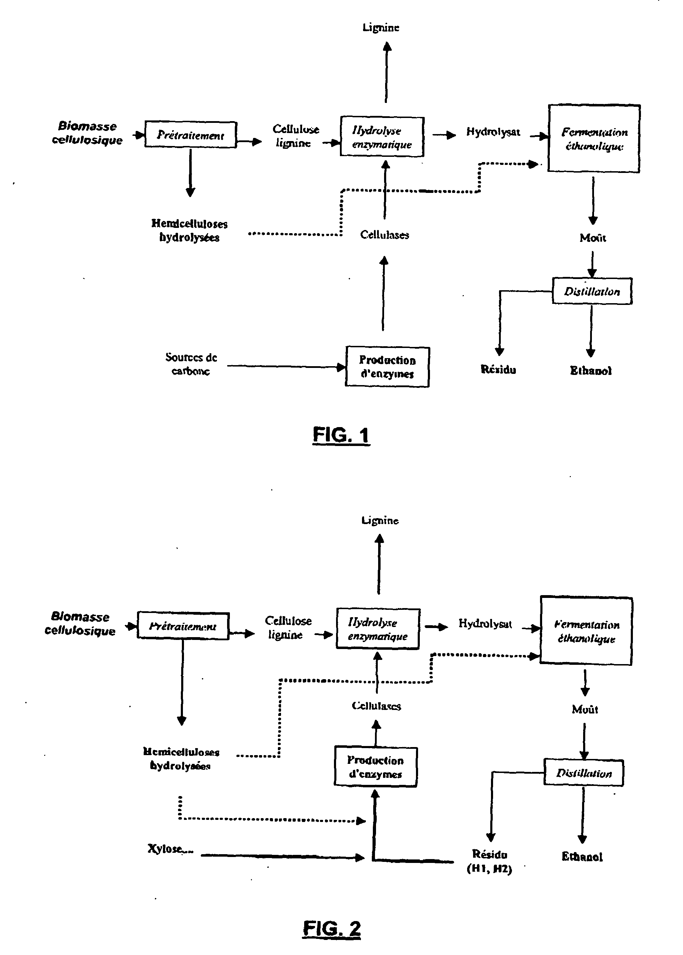

Le procédé de transformation des matériaux ligno-cellulosiques en éthanol (voir Figure 1) comprend une étape de prétraitement physico-chimique, suivie d'une étape d'hydrolyse enzymatique ou chimique, d'une étape de fermentation éthanolique des sucres libérés et d'une étape de récupération de l'éthanol.

L'étape de prétraitement a pour objet de libérer les sucres contenus dans les hémicelluloses sous forme de monomères, essentiellement des pentoses, comme le xylose et l'arabinose, et des hexoses, comme le galactose, le mannose et le glucose, et d'améliorer l'accessibilité de la cellulose engluée dans la matrice de lignine et hémicelluloses. De nombreuses technologies existent : cuissons acides, cuissons alcalines, explosion à la vapeur, procédés organosolv, etc. L'efficacité du prétraitement se mesure par le taux de récupération des hémicelluloses et par la susceptibilité à l'hydrolyse du résidu cellulosique. Les prétraitements acide en condition douce et par explosion à la vapeur sont les mieux adaptés. Ils permettent une récupération totale des pentoses et une bonne accessibilité de la cellulose à l'hydrolyse.

Le résidu cellulosique est hydrolysé, soit par voie acide, soit par voie enzymatique avec l'utilisation d'enzymes cellulolytiques et/ou hémicellulolytiques. Des microorganismes, comme les champignons appartenant aux genres *Trichoderma, Aspergillus, Penicillium ou Schizophyllum,* ou les bactéries anaérobies appartenant par exemple au genre *Clostridium,* produisent ces enzymes, contenant notamment les cellulases et les xylanases, adaptées à l'hydrolyse totale des polymères constituant les végétaux.

La voie acide, effectuée à l'aide d'acide fort, acide sulfurique notamment, est efficace mais requiert d'importantes quantités de produits chimiques (acide puis base pour la neutralisation). L'hydrolyse enzymatique ne présente pas cet inconvénient ; elle s'effectue par ailleurs dans des conditions douces et est efficace. Par contre, le coût des enzymes reste très élevé. De ce fait, beaucoup de travaux ont été conduits pour réduire ce coût : l'augmentation de la production d'enzymes d'abord, en sélectionnant les souches hyperproductrices et en améliorant les procédés de fermentation, la diminution de la quantité d'enzymes en hydrolyse ensuite, en optimisant la phase de prétraitement ou en améliorant l'activité spécifique de ces enzymes. Au cours de la dernière décennie les principaux travaux se sont attachés à comprendre les mécanismes d'action des cellulases et d'expression des enzymes afin de faire excréter le complexe enzymatique le plus approprié à l'hydrolyse des substrats ligno-cellulosiques en modifiant les souches avec les outils de biologie moléculaire.

Le microorganisme le plus utilisé pour la production de cellulases est le champignon *Trichoderma reesei.* Les souches sauvages ont la faculté d'excréter, en présence d'un substrat inducteur, la cellulose par exemple, le complexe enzymatique considéré comme le mieux adapté à l'hydrolyse de la cellulose. Les enzymes du complexe enzymatique contiennent trois grands types d'activités : les endoglucanases, les exoglucanases et les cellobiases. D'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques. La nature du substrat carboné a une forte influence sur la composition du complexe enzymatique. C'est le cas du xylose, qui, associé à un substrat carboné inducteur comme la cellulose ou le lactose, permet d'améliorer significativement l'activité dite xylanase.

Les techniques de génétique classique par mutation ont permis la sélection de souches de *Trichoderma reesei* hyperproductrices de cellulases telles que les souches MCG77 (Gallo - brevet US 4275 167), MCG 80 (Allen, A.L. et Andreotti, R.E., Biotechnol-Bioengi 1982, 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50). Les améliorations ont permis d'obtenir des souches hyperproductrices, moins sensibles à la répression catabolique sur sucres monomères notamment, glucose par exemple, par rapport aux souches sauvages.

Des souches recombinantes ont été obtenues à partir des souches de *Trichoderma reesei* Qm9414, RutC30, CL847 en clonant des gènes hétérologues, l'invertase *d'Aspergillus niger* par exemple pour diversifier la source de carbone nécessaire à la production de cellulases, et/ou surexprimant la cellobiase afin d'améliorer le rendement d'hydrolyse enzymatique, les cellobiases étant considérées comme les enzymes limitantes dans la réaction. Ces souches ont conservé leur hyperproductivité et leur aptitude à être cultivées en fermenteur.

Le procédé de production de cellulases par *Trichoderma reesei* a fait l'objet d'améliorations importantes en vue de l'extrapolation à l'échelle industrielle.

Pour obtenir de bonnes productivités en enzymes, il est nécessaire d'apporter une source de carbone rapidement assimilable pour la croissance de *Trichoderma reesei* et un substrat inducteur qui permette l'expression des cellulases et la sécrétion dans le milieu de culture. La cellulose peut jouer ces deux rôles ; cependant, elle est difficile d'utilisation au stade industriel et elle a été remplacée par des sources de carbone solubles, glucose, xylose ou lactose, le lactose jouant également le rôle de substrat inducteur. D'autres sucres solubles comme le cellobiose et le sophorose ont été décrits comme inducteurs, mais ils sont trop onéreux pour être utilisés au stade industriel. On a cependant constaté que les productions de cellulases par *Trichoderma reesei,* avec des substrats solubles, sont très inférieures à celles obtenues sur cellulose en "batch". Ceci est dû à l'effet répresseur des sucres facilement assimilables, à forte concentration. L'alimentation en continu des substrats carbonés solubles a permis de lever la répression catabolique en limitant la concentration résiduelle dans les cultures et en optimisant la quantité de sucre permettant d'obtenir un meilleur rendement et une meilleure productivité enzymatique (voir le brevet FR-B-2 555 603).

Le lactose reste, dans un procédé de production industriel d'enzymes cellulolytiques, un des substrats les plus appropriés et l'un des moins chers ; il reste néanmoins coûteux et représente environ un tiers du prix de revient des enzymes. Malgré tous les progrès réalisés, le coût des enzymes reste un poste important dans la transformation de la biomasse cellulosique en éthanol, de 30 à 50 % ; de plus, dans le cas de l'utilisation de lactose comme source de carbone pour la production de cellulases, le procédé est dépendant d'une source de carbone extérieure. De ce fait l'utilisation de substrats carbonés issus de la filière, les hémicelluloses hydrolysées par exemple, est une voie de progrès importante si la source de carbone inductrice est facilement disponible.

L'article de David J.Gregg et John N. Saddler paru dans la revue *Biotechnology and Bioengineering, Vol.51, pp375-383* (1996) décrit un procédé de production d'éthanol à partir de substrats lignocellulosiques dans lequel les enzymes utilisées pour l'hydrolyse sont recyclées plusieurs fois dans le but de minimiser les coûts.

L'article de K.Jun-suk et al paru dans J.Microbiol. Biotechnol. (1999), 9 (3), 297-302 décrit un procédé de production d'éthanol à partir de biomasses lignocellulosique dans lequel les étapes d'hydrolyse enzymatique et de fermentation éthanolique se font simultanément dans un même réacteur. Les levures et enzymes utilisées dans ce procédé dont recyclées à la fin de chaque cycle pour augmenter la production d'éthanol La séparation des enzymes et levures se fait en utilisant deux membranes différentes.

L'article de A.R.Navarro et al. publié dans Waste Management 20 (2000)581-585 décrit un procédé de bio-concentration de vinasses issues de la fermentation alcoolique de mélasses de cannes à sucre. Ces résidus sont concentrés pour limiter la quantité de déchets produits.

Le brevet US-5,98,233 décrit un procédé de fabrication d'un complexe enzymatique riche en xylanase, utilisant comme inducteur et source de carbone un résidu de distillerie de grains prétraité. Le complexe enzymatique produit est utilisé comme additif alimentaire dans l'alimentation animale.

### Description détaillée de l'invention

La présente invention concerne l'utilisation du résidu obtenu après fermentation éthanolique des sucres monomères des hydrolysats enzymatiques de biomasse cellulosique, comme source de carbone inductrice pour la production d'enzymes cellulolytiques et/ou hémicellulolytiques avec des souches de champignon cellulolytique, celles appartenant au genre *Trichoderma reesei*, dans le cadre de la production d'éthanol à partir de matériaux cellulosiques ou ligno-cellulosiques. La source de carbone principale peut être un sucre soluble industriel, par exemple le glucose, le lactose ou le xylose, ou un extrait de la fraction hémicellulosique sous forme de monomères provenant de la biomasse prétraitée. Le résidu peut aussi être utilisé aussi comme source de carbone totale, c'est-à-dire pour la croissance du microorganisme et l'induction du système d'expression. Cette source de carbone est utilisable par les souches améliorées génétiquement et notamment les souches recombinantes.

Un objet de l'invention est de proposer une source de carbone inductrice ou totale facilement disponible, permettant de produire des enzymes cellulolytiques et/ou hémicellulolytiques aux activités appropriées à l'hydrolyse de la biomasse cellulosique. Le schéma de production d'enzymes cellulolytiques et/ou hémicellulolytiques à partir de résidus de fermentation éthanolique selon l'invention est représenté à la Figure 2. Cette invention permet par ailleurs la valorisation en interne de coproduits non valorisables en éthanol.

Une première étape de prétraitement de la biomasse est réalisée pour améliorer la susceptibilité à l'hydrolyse enzymatique de la fraction cellulosique et hydrolyser la fraction hémicellulosique. La méthode la plus appropriée est l'explosion à la vapeur en condition acide. Dans les conditions optimales, de 150 à 250 °C pendant quelques minutes, elle transforme les hémicelluloses en monomères en minimisant les pertes, en furfural notamment, le xylose étant le sucre majoritaire. Les sucres libérés peuvent être extraits par lavage en phase aqueuse ; le résidu solide de l'extraction ne contient alors que de la cellulose et de la lignine. Après extraction, les sucres libérés sont utilisés pour la production d'enzymes comme source de carbone principale ou valorisés en éthanol par fermentation avec les souches appropriées.

La fraction cellulosique, débarrassée ou non de la fraction hémicellulosique hydrolysée et le cas échéant de la lignine, est hydrolysée par les enzymes cellulolytiques et/ou hémicellulolytiques produites par les souches spécialisées, les cellulases de *Trichoderma reesei* étant les plus efficaces et les plus appropriées lorsque les substrats carbonés sont issus de la biomasse cellulosique ou ligno-cellulosique. Le matériau à hydrolyser est mis en suspension en phase aqueuse à raison de 6 à 25 % de matière sèche, de préférence 10 à 20 %, le pH est ajusté entre 4 et 5,5, de préférence entre 4,8 et 5,2 et la température entre 40 et 60 °C, de préférence entre 45 et 50 °C. La réaction d'hydrolyse est démarrée par ajout des cellulases ; la quantité habituellement utilisée est de 10 à 30 mg de protéines excrétées par gramme de substrat prétraité. La réaction dure généralement de 15 à 48 heures selon l'efficacité du prétraitement, la composition du mélange de cellulases et la quantité d'enzymes ajoutées. La réaction est suivie par dosage des sucres libérés, notamment le glucose. La solution sucrée est séparée de la fraction solide non hydrolysée, essentiellement constituée de lignine, par filtration ou centrifugation ; elle est utilisée pour la fermentation éthanolique. Lorsque la fraction cellulosique a été débarrassée des hémicelluloses hydrolysées à l'étape de traitement, le glucose est le sucre majoritaire retiré dans cette étape. Le résidu de la fermentation éthanolique, après séparation de l'éthanol, est utilisé comme source de carbone inductrice ou comme source de carbone principale pour la production d'enzymes. La concentration de ce résidu est ajustée pour obtenir la concentration en source de carbone la mieux adaptée au procédé de production d'enzymes cellulolytiques et/ou hémicellulolytiques.

En général, l'éthanol est séparé du moût de fermentation par distillation et le résidu est constitué des vinasses de distillation.

Les souches utilisées pour la production d'enzymes cellulolytiques et/ou hémicellulolytiques sont des souches de champignons appartenant aux genres *Trichoderma*, *Aspergillus, Penicillium* ou *Schizophyllum,* de préférence appartenant à l'espèce *Trichoderma reesei.* Les souches industrielles les plus performantes sont les souches appartenant à l'espèce *Trichoderma reesei,* modifiées pour améliorer les enzymes cellulolytiques et/ou hémicellulolytiques par les procédés de mutation-sélection, comme par exemple la souche IFP CL847 (brevet français FR-B-2 555 803) ; les souches améliorées par les techniques de recombinaison génétique peuvent être également utilisées.

Ces souches sont cultivées en fermenteurs agités et aérés dans des conditions compatibles avec leur croissance et la production des enzymes. Selon sa nature, le substrat carboné choisi pour l'obtention de la biomasse est introduit dans le fermenteur avant stérilisation ou est stérilisé séparément et introduit dans le fermenteur après stérilisation de ce dernier pour avoir une concentration initiale de 20 à 35 g.L⁻¹ ; la source inductrice peut ne pas être ajoutée dans cette phase. Une solution aqueuse contenant le substrat choisi pour la production des enzymes est préparée à la concentration de 200-250 g.L⁻¹ ; cette solution doit contenir le substrat inducteur. Elle est injectée après l'épuisement du substrat initial de façon à apporter une quantité optimisée, comprise entre 35 et 45 mg.g⁻¹ de cellules ("fed batch"). La concentration résiduelle en sucre dans le milieu de culture est inférieure à 1 g.L⁻¹ pendant cette phase de "fed batch".

Parmi les exemples qui suivent, les Exemples 1 à 3 sont donnés à titre indicatif et les Exemples 4 et 5 illustrent l'invention.

### Exemple 1 : Production d'enzymes sur lactose

La production de cellulases est effectuée en fermenteur agité mécaniquement. Le milieu a la composition suivante : KOH 1,66 g.L⁻¹, H₃PO₄ 85 % 2 mL.L⁻¹, (NH4)₂SO₄ 2,8 g.L⁻¹, MgSO₄, 7 H₂O 0,6 g.L⁻¹, CaCL₂ 0,6 g.L⁻¹, MnSO₄ 3,2 mg.L⁻¹, ZnSO₄, 7 H₂O 2,8 mg.L⁻¹, CoCl₂ 4,0 mg.L⁻¹, FeSO₄, 7 H₂O 10 mg.L⁻¹, Corn Steep 1,2 g.L⁻¹, anti-mousse 0,5 mL.L⁻¹.

Le fermenteur contenant 1,75 L de milieu minéral et 70 g de lactose est stérilisé à 120 °C, puis ensemencé avec 0,25 litre d'une préculture liquide de la souche de *Trichoderma reesei* CL847. Le milieu de la préculture, additionné de phtalate de potassium à 5 g.L⁻¹ pour contrôler les variations de pH, est identique à celui du fermenteur. La croissance du champignon en préculture est faite sur lactose, à la concentration de 30 g.L⁻¹. La croissance de l'inoculum dure de 2 à 3 jours et est effectuée entre 27 et 30 °C sur une table d'agitation.

Après 46 heures de croissance, le substrat initial du fermenteur est épuisé et la solution de lactose à 250 g.L⁻¹ est injectée en continu au débit de 4,5 mL.h⁻¹ jusqu'à 142 heures.

La température est régulée à 27 °C pendant la phase de croissance de la biomasse, puis à 25 °C jusqu'à la fin de la culture. Le pH est régulé à 5 pendant la phase de croissance, puis à 4 jusqu'à la fin de la culture par addition d'une solution d'ammoniaque qui apporte l'azote nécessaire à la synthèse des protéines excrétées. La teneur en oxygène dissous est maintenue au-dessus de 15 à 20 % par action sur l'aération et l'agitation.

La production d'enzymes est suivie par le dosage des protéines extracellulaires par la méthode de Folin (Lowry), après séparation du mycélium par filtration ou centrifugation. Les activités cellulolytiques sont déterminées par la méthode de l'activité papier filtre (UPF : unité papier filtre) pour une activité globale et l'activité cellobiase, activité considérée comme limitante du processus d'hydrolyse enzymatique de la biomasse lignocellulosique. L'activité UPF est mesurée sur papier Whatman n° 1 à la concentration initiale de 50 g.L⁻¹ ; on détermine la prise d'essai de la solution enzymatique à analyser qui libère l'équivalent de 2 g.L⁻¹ de glucose (dosage colorimétrique) en 60 minutes. L'activité cellobiase est mesurée sur cellobiose à la concentration de 20 mM ; on détermine la prise d'essai qui libère 0,5 g.L⁻¹ de glucose (dosage enzymatique) en 30 minutes.

Les activités en U.mL⁻¹ sont exprimées en micromoles de glucose libérées par minute et par millilitre de solution enzymatique.

Les déterminations analytiques sur le moût final donnent les résultats suivants :

| | |
|---|---|
| Substrat consommé g.L⁻¹ | 79,6 |
| Biomasse g.L⁻¹ | 13,5 |
| Protéines mg.mL⁻¹ | 37,8 |
| UPF U.mL⁻¹ | 22,1 |
| Cellobiases U.mL⁻¹ | 25,2 |

### Exemple 2 : Production d'enzymes sur xylose

La production d'enzymes est menée dans les mêmes conditions que dans l'Exemple 1.

Le fermenteur contenant 1,75 L de milieu minéral avec 40 g de xylose pur est ensemencé avec 0,25 litre d'une préculture liquide de la souche de *Trichoderma reesei* CL847. Le substrat carboné de la préculture est le glucose à la concentration de 20 g.L⁻¹ Après 27 heures de croissance, après l'épuisement du substrat initial, la solution de xylose à 200 g.L⁻¹ est injectée en continu au débit de 5 mL.h⁻¹ jusqu'à 164 heures.

Les déterminations analytiques sur le moût final donnent les résultats suivants :

| | |
|---|---|
| Substrat consommé g.L⁻¹ | 63,7 |
| Biomasse g.L⁻¹ | 15,3 |
| Protéines mg.mL⁻¹ | 6,1 |
| UPF U.mL⁻¹ | 1,7 |
| Cellobiases U.mL⁻¹ | 1,9 |

### Exemple 3 : Production d'enzymes sur un mélange lactose-xylose à 25-75 % respectivement

Dans cet exemple, le lactose est utilisé comme substrat inducteur et injecté uniquement dans la phase de production d'enzymes, après l'épuisement du xylose initial.

La fermentation est conduite dans les mêmes conditions que dans l'Exemple 2. La solution de substrat injectée pour la production d'enzymes est remplacée par un mélange de lactose et de xylose à la concentration de 200 g.L⁻¹, la concentration en lactose étant de 50 g.L⁻¹. L'injection est stoppée à 164 heures.

Les déterminations analytiques sur le moût final donnent les résultats suivants :

| | |
|---|---|
| Substrat consommé g.L⁻¹ | 62,7 |
| Biomasse g.L⁻¹ | 11,9 |
| Protéines mg.mL⁻¹ | 34,2 |
| UPF U.mL⁻¹ | 10,1 |
| Cellobiases U.mL⁻¹ | 8,7 |

### Exemple 4 : Production d'enzymes sur xylose en utilisant le résidu produit par fermentation éthanolique d'un hvdrolvsat enzymatique partiel d'un matériau cellulosique traité et dépentosé, comme source inductrice

La fermentation est conduite dans les mêmes conditions que dans l'Exemple 2. La production de biomasse est faite en "batch" avec 20 g.L⁻¹ de xylose. La production d'enzymes est faite en "fed batch" avec une solution préparée à partir d'un résidu dans lequel on ajoute du xylose. La solution de résidu, référencée H1, est préparée de la façon suivante :

6 kg de matériau solide humide, obtenu après traitement de paille de blé par explosion à la vapeur en condition acide et extraction des pentoses, soit 1,4 kg de matière sèche traitée et lavée, sont repris dans 0,75 litre de tampon acétate de sodium 0,5 M à pH 4,8 et 0,75 litre d'une solution de cellulases commerciales, soit 30 g de protéines. Après une hydrolyse enzymatique de 24 heures, réalisée dans un réacteur agité mécaniquement et maintenu à 50 °C. La suspension est séparée par centrifugation. La fraction liquide récupérée contient 92 g.L⁻¹ de glucose, 10,3 g.L⁻¹ de xylose, 1.0 g.L⁻¹ d'arabinose. Une fermentation éthanolique est faite sur cette fraction par *Saccharomyces cerevisiae*. L'inoculum, d'un volume de 0,4 L, est préparé en fiole agitée sur glucose avec une levure de boulangerie. Le glucose de l'hydrolysat est totalement fermenté en éthanol. Le moût obtenu est débarrassé des protéines et de la levure par chauffage à 100 °C et centrifugation. Après concentration sous vide, on obtient 0,85 L de la vinasse H1 débarrassée de l'éthanol. La solution de substrat, d'un volume de 1,5 L, utilisée pour la production d'enzyme, est préparée à partir de cette vinasse avec ajout de xylose pour obtenir une concentration finale en substrat carboné de 200 g.L⁻¹. La fermentation est conduite dans les mêmes conditions que pour les Exemples 1 à 3. La fermentation est stoppée à 136 heures.

Les déterminations analytiques sur le moût final donnent les résultats indiqués dans le tableau ci-dessous. Les protéines sont précipitées par l'acide trichloracétique afin d'éviter la réaction colorée indésirable liée aux impuretés du résidu.

| | |
|---|---|
| Substrat consommé g.L⁻¹ | 59,0 |
| Biomasse g.L⁻¹ | 12,0 |
| Protéines mg.mL⁻¹ | 33,6 |
| UPF U.mL⁻¹ | 10,5 |
| Cellobiases U.mL⁻¹ | 4,5 |

### Exemple 5 : Production d'enzymes sur xylose en utilisant le résidu produit par fermentation éthanolique d'un hvdrolvsat poussé d'un matériau cellulosique traité et dépentosé, comme source inductrice

Comme dans l'Exemple 4, la production de biomasse est faite en "batch" avec 20 g.L⁻¹ de xylose. La production d'enzymes est faite en "fed batch" avec une solution de substrat préparée à partir de vinasses dans lesquelles on ajoute du xylose. Dans le cas présent, la solution de vinasses, référencée H2, est préparée de la même façon que pour H1 mais l'hydrolyse est prolongée jusqu'à 48 heures avec un ajout d'enzymes, identique au premier ajout, après 24 heures d'hydrolyse. L'hydrolysat contient 110,8 g.L⁻¹ de glucose, 11,8 g.L⁻¹ de xylose. La vinasse H2, après élimination de l'éthanol et concentration, est utilisée pour préparer 1,5 L d'une solution de substrat à 200 g.L⁻¹ en sucres totaux. La fermentation est conduite dans les mêmes conditions que pour l'Exemple 4 ; la fermentation est stoppée à 158 heures.

Les déterminations analytiques sur le moût final donnent les résultats suivants :

| | |
|---|---|
| Substrat consommé g.L⁻¹ | 62,5 |
| Biomasse g.L⁻¹ | 14,5 |
| Protéines mg.mL⁻¹ | 28,0 |
| UPF U.mL⁻¹ | 10,0 |
| Cellobiases U.mL⁻¹ | 4,7 |

## Revendications

1. Procédé de production d'éthanol à partir de matériaux cellulosiques ou ligno-cellulosiques comprenant les étapes suivantes :
1) le prétraitement chimique et/ou physique d'un substrat cellulosique ou lignocellulosique ;
2) l'hydrolyse enzymatique du substrat prétraité utilisant des enzymes cellulolytiques produites par les champignons appartenant à l'espèce *Trichoderma reesei* ;
3) la fermentation éthanolique par un micro-organisme alcooligène approprié de l'hydrolysat issu de l'étape (2) et obtention d'un moût de fermentation ; et
4) la séparation du micro-organisme alcooligène utilisé dans l'étape (3), la séparation/purification de l'éthanol et l'obtention d'une phase aqueuse constituant un résidu,
dans lequel le résidu constitué des vinasses de distillation est utilisé, après séparation de l'éthanol, comme source de carbone inductrice ou totale pour la production desdites enzymes cellulolytiques .

2. Procédé selon la revendication 1 dans lequel ledit résidu est utilisé comme source de carbone pour la croissance du microorganisme cellulolytique et la production d'enzymes cellulolytiques.

3. Procédé selon la revendication 1 ou 2 dans lequel le résidu est utilisé comme source de carbone inductrice en complément d'autres sources de carbone utilisées pour la croissance du microorganisme cellulolytique.

4. Procédé selon la revendication 1 ou 2 dans lequel le résidu est utilisé comme unique source de carbone pour la croissance du microorganisme cellulolytique et la production d'enzymes cellulolytiques .

5. Procédé selon l'une des revendications 1 à 4 dans lequel les matériaux cellulosiques ou ligno-cellulosiques sont choisis parmi les pailles, le bois, les cultures forestières, les résidus de plantes alcooligènes, sucrières et céréalières, les résidus de l'industrie papetière et les produits de transformation des matériaux cellulosiques et lignocellulosiques.

## Claims

1. A process for producing ethanol from cellulosic or ligno-cellulosic materials comprising the following steps:
1) chemical and/or physical pre-treatment of a cellulosic or lignocellulosic substrate;
2) enzymatic hydrolysis of a pre-treated substrate using cellulolytic enzymes produced by fungi belonging to the species *Trichoderma reesei*.;
3) ethanolic fermentation by a suitable alcohologenic microorganism of the hydrolysate from step (2) and production of a fermentation must; and
4) separating the alcohologenic microorganism used in step (3), separating/purifying the ethanol and obtaining an aqueous phase constituting a residue;
wherein the residue constituted by distillation slops is used, after ethanol separation, as a source of carbon for growth of the cellulolytic microorganism and the production of cellulolytic and/or hemicellulolytic enzymes.

2. A process according to claim 1, in which said residue is used as a source of carbon for growth of the cellulolytic microorganism and the production of cellulolytic enzymes.

3. A process according to claim 1 or 2 , in which the residue is used as a source of inducing carbon as a complement to other sources of carbon used for growth of the cellulolytic microorganism.

4. A process according to claim 1 or claim 2, in which the residue is used as a unique source of carbon for growth of the cellulolytic microorganism and production of cellulolytic enzymes.

5. A process according to one of claims 1 to 4, in which the cellulosic or ligno-cellulosic materials are selected from straw, wood, forest culture, alcohologenic residues from plants, sugar refineries and cereal producers, residues from the paper industry and products for transforming cellulosic and ligno-cellulosic materials.

## Patentansprüche

1. Verfahren zur Herstellung von Ethanol aus zellulosehaltigen oder lignozellulosehaltigen Materialien, das die folgenden Schritte umfasst:
1) chemisches und/oder physikalisches Vorbehandeln eines zellulosehaltigen oder lignnzellulosehaltigen Substrats;
2) enzymatisches Hydrolysieren des vorbehandelten Substrats unter Verwendung der zellulosespaltenden Enzyme, die von Pilzen produziert werden, die zur Spezies *Trichoderma reesei* gehören;
3) ethanolisches Fermentieren des Hydrolysats aus Schritt (2) durch einen geeigneten alkoholproduzierenden Mikroorganismus und Erhalten einer Fermentationsmaische; und
4) Abtrennen des alkoholproduzierenden Mikroorganismus, der in Schritt (3) verwendet wurde, Abtrennen/Reinigen des Ethanols und Erhalten einer wässrigen Phase, die einen Rückstand bildet;
wobei der Rückstand, der aus Destillationsschlempen besteht, nach dem Abtrennen des Ethanols, als induzierende oder gesamte Kohlenstoffquelle zur Produktion der zellulosespaltenden Enzyme verwendet wird.

2. Verfahren nach Anspruch 1, wobei der Rückstand als Kohlenstoffquelle für das Wachstum des zellulosespaltenden Mikroorganismus und zur Produktion von zellulosespaltenden Enzymen verwendet wird.

3. Verfahren nach Anspruch 1 oder 2, wobei der Rückstand als induzierende Kohlenstoffquelle zusätzlich zu anderen Kohlenstoffquellen verwendet wird, die für das Wachstum des zellulosespaltenden Mikroorganismus verwendet werden.

4. Verfahren nach Anspruch 1 oder 2, wobei der Rückstand als einzige Kohlenstoffquelle für das Wachstum des zellulosespaltenden
Mikroorganismus und zur Produktion von zellulosespaltenden Enzymen verwendet wird.

5. Verfahren nach einem der Ansprüche 1 bis 4, wobei die zellulosehaltigen oder lignozellulosehaltigen Materialien ausgewählt sind aus Stroh, Holz, Forstkulturen, Rückständen von alkoholproduzierenden Pflanzen, Zuckerpflanzen und Getreidepflanzen, Rückständen der Papierindustrie und Transformationsprodukten der zellulosehaltigen und lignozellulosehaltigen Materialien.
